# EUROPEAN PATENT SPECIFICATION

(11) **EP 3 954 359 B1**
(45) Date of publication and mention of the grant of the patent: **11.11.2026**
(21) Application number: 19924158.9
(22) Date of filing: 11.04.2019
(51) Int. Cl.: A61K 8/97, A61K 8/9789, A61K 8/44, A61K 8/60, A61K 8/73, A61Q 19/00, A61Q 19/08, A61K 8/92

(54) **TOPICAL COSMETIC COMPOSITION AND USES THEREOF**
TOPISCHE KOSMETISCHE ZUSAMMENSETZUNG UND VERWENDUNGEN DAVON
COMPOSITION COSMÉTIQUE TOPIQUE ET SES UTILISATIONS

(43) Date of publication of application: 16.02.2022
(73) Proprietor: Natura Cosméticos S.A., 05106-000 São Paulo SP (BR)
(72) Inventor: PEREIRA SANTOS, Camila, 05106-000 São Paulo - SP (BR); ZIMBARDI, Daniela, 05106-000 São Paulo - SP (BR); DE MIRANDA CHAVES VASQUEZ PINTO, Luciana, 05106-000 São Paulo - SP (BR); DO NASCIMENTO, Selma, 05106-000 São Paulo - SP (BR); ARANDAS MONTEIRO E SILVA, Silas, 05106-000 São Paulo - SP (BR); LIMA GUSMÃO, Edjane, 05106-000 São Paulo - SP (BR); ARRUDA COSTA, Andrea, 05106-000 São Paulo - SP (BR)
(74) Representative: Simmons & Simmons
(86) International application number: PCT/BR2019/050134
(87) International publication number: WO 2020/206515

(56) References cited:
- WO-A1-2016/029285
- WO-A1-2017/193186
- WO-A1-2017/193186
- WO-A1-2019/023772
- WO-A1-2019/023774
- WO-A1-2019/023774
- CN-A- 103 462 881
- CN-A- 106 580 860
- CN-A- 107 184 486
- CN-A- 108 434 033
- CN-A- 108 542 801
- CN-A- 108 904 334
- JP-A- H0 853 360
- US-A1- 2013 302 389
- CARRANCA FILMES: "Natura Nhandiroba ", NATURA BERN ESTAR BEM- NHANDIROBA: FEVILLEA TRILOBATA. - NATURA, 28 February 2018 (2018-02-28), XP055861157, Retrieved from the Internet <URL:https://vimeo.com/257970896> [retrieved on 20191126]

## Description

### Field of the invention

The present invention describes a topical cosmetic composition and the uses thereof to provide improved hydration and preserve the natural and healthy balance of the skin microbiota and correlated method. The present invention is within the field of cosmetic science, more precisely it refers to cosmetic preparations for active hydration and preservation of the skin microbiota.

### Background of the invention

The skin is the largest and most visible organ in the human body and plays a fundamental role in maintaining life. It performs multiple vital functions to the body, and its role in protecting the body against fluid loss and preventing the penetration of foreign substances into the body must be highlighted.

The skin is also an ecosystem composed of about 2 m² diverse habitats, with abundant folds, invaginations and specialized niches that can support a wide range of microorganisms such as bacteria, fungi, mites and viruses. These microorganisms comprise the so-called skin flora and, like the skin itself, they play a key protective role, as they contribute to the skin integrity and immuno homeostasis.

Currently, both variations that change the composition of the skin layers and variations in the balance of the skin flora are known to cause damage to the skin which, in addition to causing discomfort, may also not be visually pleasing. Thus, there is a need to develop topical cosmetic compositions that contribute, at the same time, to the regulation of the skin morphology and the preservation of the balance of the microbial flora.

Stratum corneum is the skin region that contacts the outside environment, being the outer layer of the epidermis. Characteristics of the stratum corneum are critically related to its composition, being represented by proteins (75-80%), lipids (5-15%) and other constituents (5-10%). When these components are not properly balanced, the skin ability to hold water is reduced and the skin becomes more susceptible to the influence of environmental factors, causing an impaired barrier.

Endogenous factors due to aging or, for example, hormonal influence, and exogenous factors such as chronic exposure of the skin to dry environments, UV rays, wind, chemicals, etc., are factors that interfere with the skin ability to hold water. As a result, poorly hydrated skin (dry skin) lacks shine and flexibility, having a rough appearance and a tendency to peel off.

This type of skin requires specific care, since the lack of hydration can compromise the skin integrity.

Numerous moisturizing cosmetic compositions aim to be alternatives to solve the issue of dry skin. It is even recommended that adults use moisturizing compositions all over their skin at least once a day.

Traditional skin moisturizing products generally include ingredients that typically act through two well-known mechanisms: occlusion and wetting. Hydration by occlusion is promoted by ingredients that form a lipophilic film that makes water loss more difficult, hence increasing water retention in the stratum corneum. Wetting hydration, in turn, is promoted by ingredients that retain water from the cosmetic composition, the atmosphere and the skin, on the skin surface through a hydrophilic film formed on the stratum corneum, hence increasing water retention on the skin surface.

On the other hand, many moisturizing cosmetics for topical use do not take into account that the skin is an ecosystem having a delicate balance between the host and microorganisms, therefore, they contain ingredients that hinder the development of beneficial microorganisms for healthy skin.

The skin is a niche for several microorganisms such as viruses, bacteria, fungi and mites that cover the skin surface and reside in the skin appendages that include hair follicles, sebaceous glands and sweat glands. These different microorganisms constitute a natural and healthy flora and provide vital functions to the body, protecting the skin from invasion of pathogenic or harmful organisms.

On the skin surface, bacteria such as those from the phylum *Proteobacteria* and from the genus *Staphylococcus* form communities that are deeply interconnected with each other and with other microorganisms. Commensal fungi such as *Malassezia spp.* grow both as branched filamentous hyphae and as individual cells. Skin mites, such as *Demodex folliculo* rum and *Demodex brevis* are some of the smallest arthropods and live inside or near the hair follicles.

When there is an imbalance of the natural and healthy skin flora, the skin can suffer the invasion or manifestation of pathogenic bacteria, which can cause stress and inflammation, redness, itching, scarring, erythema and other reactions, leading to loss of the original skin radiance, while at the same time causes more skin problems and even skin aging.

The flora imbalance can be caused by the use of topical cosmetic compositions that contain ingredients that are harmful to the natural and healthy balance of the skin microbiota. Such compositions, therefore, can result in skin disorders or infections.

It is difficult to modify a cosmetic composition without changing its ability to satisfactorily play its key role, for example, hydration. On the other hand, it is a necessary task for the improvement of cosmetic compositions.

Thus, there is a need to develop cosmetic compositions that favorably change the regulation of the microbiological flora balance, while also making the skin smooth, shiny and elastic through skin hydration.

In the search for the state of the art in scientific and patent literature, the following documents dealing with the subject matter were found:
Document CN103462881 describes a cosmetic composition intended to promote the balance of skin microbiota.

The document comments on the need to maintain the skin microbiota to avoid skin damage, for example, growth of pathogenic microorganisms, due to an unbalanced microbiota. In fact, the document already describes that this need is much debated in modern cosmetics, being one of the factors that led to the replacement of antibiotics, hormones and other compounds that can be harmful to the balance of the natural microbiota when present in topical cosmetic compositions.

In detail, the composition described in the Chinese document has the following components: glycerin, butylene glycol, hamamelis extract, allantoin, EDTA trisodium, glycerin, purslane extract, alpha-glucan, oligosaccharide, xylitol, wicker extract, fructooligosaccharide, vitamin B5, Asian centella extract, rice bran extract, yeast extract, betaine, polyglutamic acid, sodium hyaluronate, beta-glucan, trehalose, potassium glycyrrhizinate, urea, sodium taurate, radish root fermentation product filter liquor and leuconostoc.

Document BR 11 2018 073287 refers to a cosmetic skin repairing composition, comprising an oil selected from *Bertholletia excelsa* or *Fevillea trilobata* and cosmetically acceptable excipients. The composition is said to provide an anti-aging treatment, including reducing wrinkles and promoting the skin regenerative capacity.

Furthermore, the Brazilian document describes that the composition acts by modulating cellular processes involved in skin repair, particularly in the mechanism of skin regeneration and healing, including FGF-2, IL-1B, IL-6, IL-8, IL- 10, IL-12, cell migration and proliferation associated with skin barrier construction and elastase activity (MMP-12).

Thus, it is understood that, in view of the state of the art found, the problem related to the need to provide cosmetic compositions that enable the production of cosmetics having improved hydration efficiency and that aid to maintain the skin flora still exists.

### Summary of the invention

Thus, the present invention is intended to solve the technical problems present in the state of the art by providing a topical cosmetic composition that causes active hydration of the skin and/or preservation of the maintenance of the skin natural and healthy microbiota.

In a first aspect, the present invention relates to a topical cosmetic composition comprising the association of *Fevillea trilobata* seed oil, trehalose, sodium hyaluronate, betaine and cosmetically acceptable excipients.

In a second aspect, the present invention refers to the use of the cosmetic composition of the first aspect to promote skin hydration to the body or face.

In a third aspect, the present invention relates to the use of the cosmetic composition of the first aspect to preserve the skin microbiota.

Furthermore, the inventive concept in common to all claimed contexts is the topical cosmetic composition.

From among the advantages of the present invention, in a non-exhaustive manner, is that it is able to:
- provide improved skin hydration;
- provide prolonged skin hydration after discontinuation of the use of the product;
- provide intelligent hydration by repairing the hydration levels according to the needs of each face region;
- preserve the skin natural and healthy microbiota;
- provide cell renewal;
- provide adhesion and cohesion between skin cells and layers;
- provide an anti-aging effect;

These and other objects of the invention will be immediately valued by those skilled in the art and by companies with interest in the segment, and will be described in sufficient detail to be reproduced in the description below.

### Brief description of the figures

In order to better define and clarify the present invention, the following figures are presented:
Figure 1 shows a graph of mean capacitance values (h).
Figure 2 shows a graph of the mean values of the percent increase in skin hydration (*%Hh*)
Figure 3 shows the facial skin water profile in a participant at time zero of the assessment.
Figure 4 shows the facial skin water profile in a participant 28 days after using the product.
Figure 5 shows the facial skin water profile three days after discontinuing the use of the product.
Figure 6 shows a graph with the count of colony-forming units in cultures of swabs collected from human skin at the following experimental times: T-14, T0, T14.
Figure 7 shows the comparison of complex I, complex II and a placebo relative to the gene expression related to active skin hydration.
Figure 8 shows the comparison of complex I, complex II and a placebo relative to the viability kinetics of *S*. *epidermidis.*
Figure 9 shows the comparison of complex I, complex II and a placebo relative to the viability kinetics of *S. aureus.*

### Detailed description of the invention

The present invention describes a topical cosmetic composition and uses thereof in skin hydration and/or preservation of maintenance of the skin microbiota.

In the present invention the term "hydration" can be designated as "active hydration", "bioactive hydration", "biological hydration", "biodynamic hydration", "intelligent hydration". The hydration described herein stimulates the endogenous and natural mechanism of the skin, being able to maintain hydration at optimal levels for longer periods.

The hydration of the present invention is conceived by increasing the skin water content in different regions of the epithelial tissue.

In the present invention, the term "maintenance of the skin microbiota" can also be understood as "preservation of the skin microbiota" or "microbiota balance", being defined as an ability to enable the presence of a natural and healthy skin microbiota. Furthermore, the term "skin microbiota" can also be understood as "skin flora".

The present invention differs in many ways from the state of the art. For example, the present invention uses no microorganisms or their metabolite products. Furthermore, the state of the art discloses compositions containing components different from those of the present invention, which makes it impossible for them to provide the same technical effect.

The present invention is a cosmetic composition for hydration and/or preservation of the skin microbiota. The present invention uses a *Fevillea* crude oil among its active ingredients, which is essential to trigger the technical effects of the present invention, when combined with other ingredients present in the cosmetic composition.

In a first object, the invention relates to a cosmetic composition comprising the association of *Fevillea trilobata* seed crude oil, trehalose, sodium hyaluronate, betaine and cosmetically acceptable excipients.

*Fevillea* crude oil is a product from the Brazilian biodiversity extracted from the seeds of *Fevillea trilobata* plant. It is an oil rich in punic acid or conjugated trienes (conjugated C18:3), beta-sitosterol, gamma-tocopherol and it further contains bioactive compounds such as alkaloids, terpenes, glycosides and tannins. On the skin, it stimulates the natural mechanisms for the production of hyaluronic acid, increasing water levels in the deeper layers of the skin. In addition, the oil also stimulates human skin cells to produce, by natural synthesis, elastin, filaggrin, ELOVL3 and claudin I.

Fevillea's ability to enhance the natural synthesis can be identified, for example, through an immunofluorescence test in an *ex vivo* skin model.

Trehalose is a biotechnological active of plant origin. The ingredient has a prebiotic function as it works to maintain and balance the skin microorganisms, helping to strengthen the skin barrier. Furthermore, it is a skin conditioning and humectant agent.

Betaine can be obtained, for example, from sugar beet. Betaine improves the skin barrier, increases the number of natural (or endogenous) molecules that retain water on the surface layers and increases hydration.

Furthermore, betaine and trehalose are osmotically active ingredients; therefore, they exhibit a high moisturizing performance potential by producing cell osmotic difference.

Sodium hyaluronate is a biotechnological active of natural origin. Its roles are the ability to increase water levels on the skin surface, acting as a skin conditioner or moisturizer.

Association of the four ingredients, *Fevillea trilobata* crude seed oil, trehalose, sodium hyaluronate and betaine is required for the purposes of the present invention, since they form an association capable of providing an active hydration of the skin, stimulating the endogenous and natural self-hydration mechanism of the skin and is also capable of preserving the skin microbiota.

The active ingredients in isolation from said association do not act on the active hydration mechanism, so they have to be combined.

Also, inclusion of this association in the topical cosmetic composition of the present invention has promoted greater effectiveness of the action of the complex.

In this case, the cosmetic composition of the present invention is capable of: (i) stimulating proper and balanced removal of dead cells from the skin surface/renewal of skin barrier cells; (ii) stimulating molecules that retain surface water such as NMF (natural moisturizing factors), mineral salts and lipids; (iii) stimulating molecules that retain water in the deeper layers of the skin (dermis) such as hyaluronic acid; and stimulating essential proteins to maintain the skin fluid-lipid balance. It has also promoted a greater ability to preserve the skin natural and healthy microbiota.

By cosmetically acceptable excipients is meant non-irritating and non-toxic substances, which are used to prepare and/or provide structure to a cosmetic form, and may or may not confer organoleptic properties on a composition such as, for example, color and odor, and may or may not provide a cosmetically desirable biological effect such as hydration. The cosmetically acceptable excipients of the present invention can be formulated in different amounts, depending on the type of characteristics envisioned in the final cosmetic product.

The cosmetically acceptable excipients in accordance with the present invention may be those known to the person skilled in the art, such as those cited in the "International Cosmetic Ingredient Dictionary & Handbook", 16th Edition.

In particular, the cosmetically acceptable excipients of the present invention can be, not exhaustively, those belonging to the classes of viscosity controllers, anti-foaming agents, emollients, skin conditioners, skin protectors, denaturants, perfumes, moisturizers, opacifiers, emulsifiers, preservatives, antioxidants, vegetable oils, emulsion stabilizers, plasticizers, chelators, buffers and colorants.

Preferably, the solvent can be water, propanediol and pentylene glycol, or a combination thereof; the anti-foaming agent can be dimethicone; the emollient can be dimethicone, vegetable oils and tocopherol, or a combination thereof; the skin conditioner can be dimethicone, pentylene glycol, glycerin, betaine, capric/caprylic triglyceride, tocopherol, sodium hyaluronate, sorbitol, sodium gluconate, or a combination thereof; the skin protector can be dimethicone, glycerin or a combination thereof; the viscosity controller can be propanediol, glycerin, betaine, carbomer, ammonium acryloyldimethyltaurate copolymer, sodium chloride, sodium sulfate or combination thereof; the denaturant can be glycerin, sodium hydroxide, eugenol or a combination thereof; the humectant can be glycerin, trehalose, betaine, sodium hyaluronate, sorbitol or a combination thereof; fragrances and perfumes can be used; opacifier can be polymethylsilsesquioxane; the emulsifier can be trilaureth-4 phosphate, polyglyceryl-2 sesqui-isostearate or combination thereof; the preservative can be phenoxyethanol or hydroxyacetophenone or a combination thereof; the antioxidant can be tocopherol, hydroxyacetophenone or combination thereof; the emulsion stabilizer can be carbomer; the plasticizer can be sorbitol; the chelator can be sodium gluconate; and the buffering agent can be sodium hydroxide; sodium carbonate or a combination thereof.

Preferably, the cosmetic composition contains from about 0.1 to about 5% by weight *Fevillea trilobata* crude seed oil, from about 0.5 to about 8% by weight trehalose, from about 0.05 to about 5% by weight sodium hyaluronate, from about 0.5 to about 10% by weight betaine and from about 70% to about 99% by weight of cosmetically acceptable excipients. More preferably, the composition comprises from about 0.1 to about 3% by weight *Fevillea trilobata* crude seed oil, from about 1 to about 4% by weight trehalose, from about 0.1 to about from 2% by weight sodium hyaluronate, from about 1 to about 5% by weight betaine and from about 86% to about 97.8% by weight of cosmetically acceptable excipients.

The cosmetic composition of the present invention is for topical use, whether on the face or body. Its cosmetic forms can be: emulsions, creams, creamy emulsions, gels, cream gels, aqua gels, acqua gels and serums.

Preferably, the cosmetic composition is an acqua gel for use on the face or body. Even more preferably, it is an acqua gel for facial use.

One embodiment of the present invention can provide hydration with a significant increase of the skin water content in different regions of the epithelial tissue.

Furthermore, the embodiment of the present invention can provide a significant increase in facial skin hydration after 28 days of home use as compared to the baseline condition.

One embodiment of the invention has provided a significant improvement of the facial skin 3 and 4 days after discontinuation of home use compared to the baseline condition, indicating maintenance of facial skin hydration for up to 4 days after discontinuation of use.

Thus, in a second aspect the invention refers to the use of the composition as defined in the first aspect to provide skin hydration to the body or face.

Preferably, the invention relates to the use of the composition to provide skin hydration to the face.

In one embodiment of the first object, the use of the composition of the present invention was not shown to lead to significant changes in the number of microorganisms, preserving the natural and healthy microbiota of the skin.

Therefore, in a third aspect, the invention relates to the use of the cosmetic compositions of the first aspect to preserve the natural skin microbiota.

### Examples - Embodiments

The examples shown herein are intended only to exemplify one of the numerous ways of carrying out the invention, without limiting its scope.

For the purpose of illustrating the technical effects of the present invention, and not to limit it, whenever the examples refer to an "investigational product" reference will be made to the preferred composition embodiment of the first aspect. The composition of the "investigational product" can be found in table 1.

**Table 1 - Composition of the investigational product**

| Ingredient | Minimum Concentration (% w/w) | Maximum Concentration (% w/w) |
|---|---|---|
| Water | qsp | qsp |
| Dimethicone | 7 | 15 |
| Pentylene glycol | 7 | 15 |
| Glycerin | 7 | 15 |
| Propanediol | 3 | 7 |
| Trehalose | 0.5 | 8 |
| Polymethylsilsesquioxane | 1 | 3 |
| Betaine | 0.5 | 10 |
| Capric/caprylic triglyceride | 1 | 3 |
| Phenoxyethanol | 0.5 | 0.8 |
| Ammonium acrylomethyldimethyltaurate copolymer | 0.4 | 2 |
| Tocopherol | 0.3 | 0.7 |
| Carbomer | 0.3 | 0.6 |
| *Fevillea trilobata* seed crude oil | 0.1 | 5 |
| Hydroxyacetophenone | 0.3 | 0.6 |
| Sodium Hyaluronate | 0.05 | 5 |
| Parfum | 0.1 | 0.3 |
| Sorbitol | 0.1 | 0.3 |
| Sodium gluconate | 0.05 | 0.15 |
| Sodium hydroxide | 0.07 | 0.15 |
| Trilaureth-4 phosphate | 0.05 | 0.1 |
| Polyglyceryl-2 sesquiisostearate | 0.04 | 0.1 |
| Citronellol | 0.005 | 0.02 |
| Alpha-isomethyl ionone | 0.002 | 0.005 |
| *Conobea scoparioides* leaf oil | 0.001 | 0.003 |

| Ingredient | Minimum Concentration (% w/w) | Maximum Concentration (% w/w) |
|---|---|---|
| Sodium carbonate | 0.0003 | 0.0006 |
| Ci 42090 | 0.00006 | 0.0001 |
| Sodium Chloride | 0.00001 | 0.00004 |
| Sodium Sulfate | 0.000004 | 0.000007 |

### Example 1 - Evaluation of skin hydration by corneometry

### Goal

This study is aimed to evaluate skin hydration after application of the investigational product on the face of the survey participants.

Hydration assessment was performed by measuring the skin capacitance using the corneometry technique.

### Methodology

### Selection of participants

The participants chosen for the study met the following criteria: age group from 18 to 60 years old; gender: female; phototype (fitzpatrick): I to V; skin physiological characteristic: dry; intact skin in the test region; agreement not to use any topical products on the test region; be a user of cosmetic products of the same category. Twenty-one participants have completed the study.

### Procedure for carrying out evaluations

Four areas measuring 2.5 cm x 4.0 cm, designated sites, were marked on the face of the survey participants - on the forehead, right malar, left malar and mentum.

After 30 minutes of acclimatization in a controlled environment at 20 ± 2°C and 50 ± 5% relative humidity, baseline measurements (prior to the home use of the investigational product) of skin capacitance at the marked sites were obtained.

New capacitance measurements were obtained after 28 days of home use of the investigational product and 3 (T31) and 4 (T32) days after discontinuation of the use of the investigational product.

During the time of home use of the investigational product, participants were asked to to fill out a usage diary, that had to be filled out every day.

Throughout the entire laboratory experiment, the climatic conditions were kept constant in accordance with the the aforementioned ranges.

### Acquisition of capacitance measurements

Capacitance measurements were performed using the Corneometer^{®} 825 probe coupled to the Multi Probe Adapter, MPA 5 (CKeletronics, Germany).

### Results

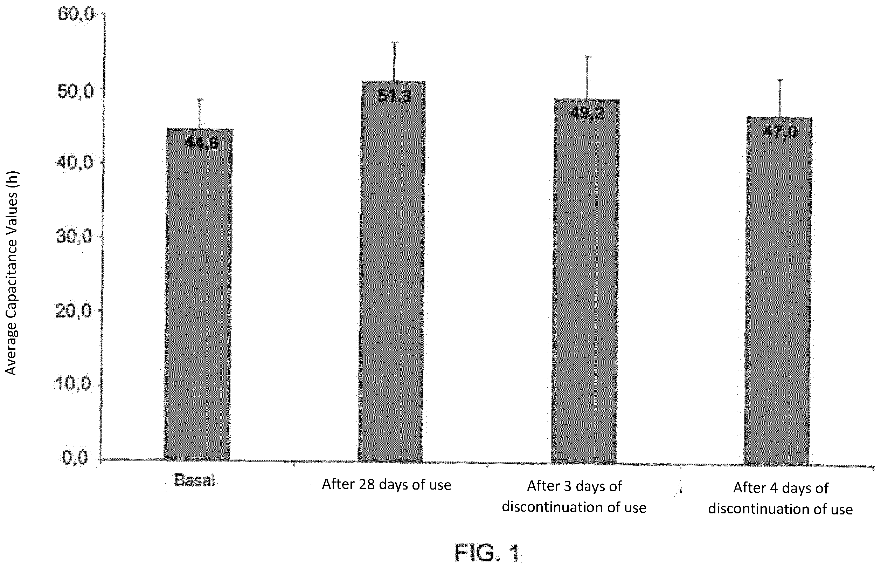

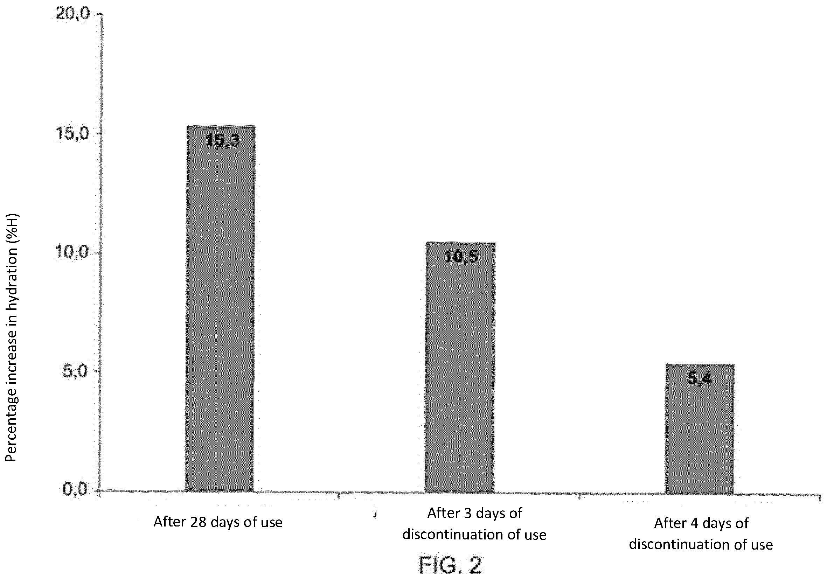

The graphs shown in Figures 1 and 2 show the mean capacitance values (h) and the percentage of increase in skin hydration (%Hₕ) obtained throughout the study, respectively.

Table 2 summarizes the statistical results of the significance of the variation in skin hydration throughout the study.

**Table 2 - Summarized data from the statistical significance of skin hydration.**

| Comparison groups | Investigational Product |
|---|---|
| ht0 vs. h28 days of use | < 0.0001 (significant |
| ht0 vs. ht3 days after discontinuation of use. | < 0.0001 (significant) |
| ht0 vs. ht4 days after discontinuation of use. | 0.0095 (significant) |

Based on the results, the following conclusions were drawn:
- the investigational product significantly increased skin hydration after 28 days of home use as compared to the baseline condition.
- the investigational product significantly increased facial skin hydration 3 and 4 days after discontinuation of home use compared to the baseline condition, indicating maintenance of facial skin hydration for up to 4 days after discontinuation of use.
- the investigational product increased the skin hydration level by up to 15.3%.
- 100% of the survey participants have shown improvement in facial skin hydration after treatment with the investigational product. Example 2 - Analysis of the water profile.

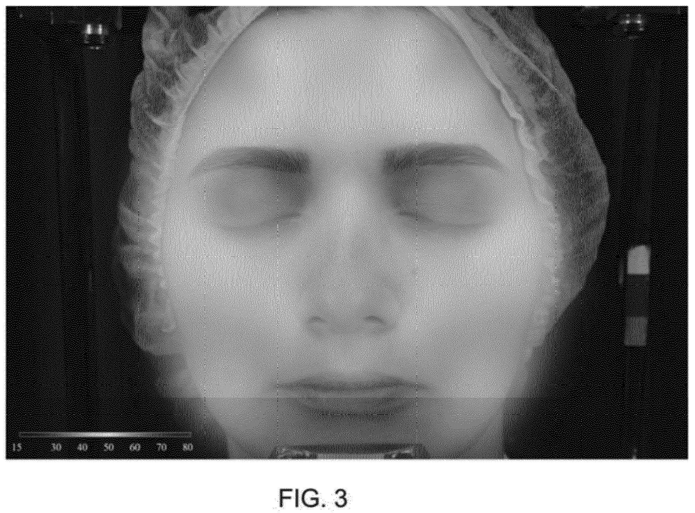

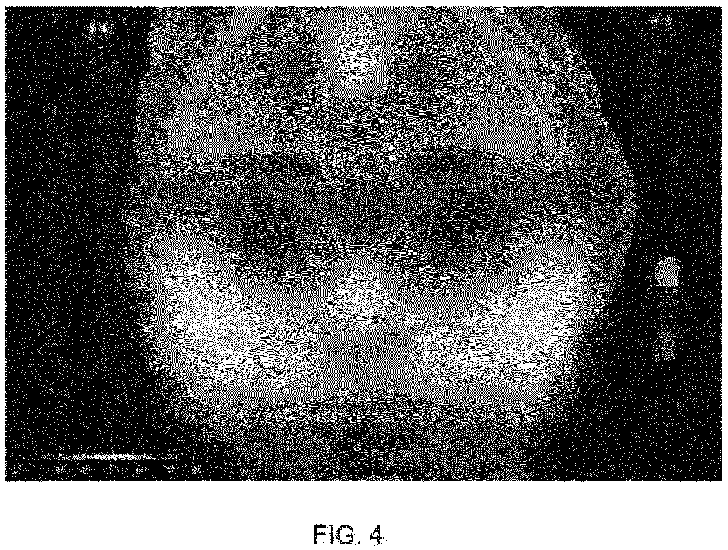

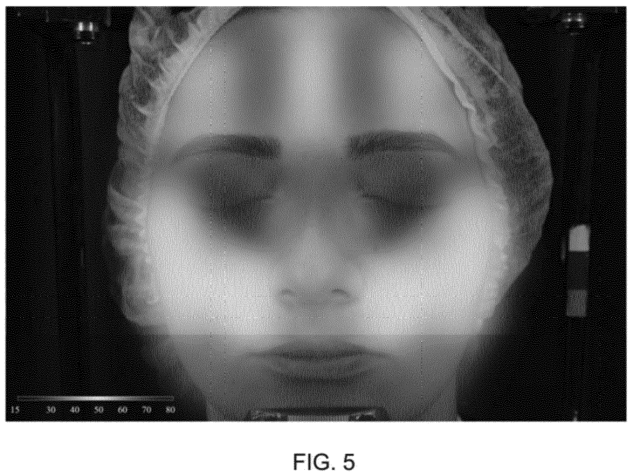

Figures 3, 4 and 5 show the increased water content in the skin at different regions of the face over a period of use of the investigational composition.

### Example 3 - Evaluation of maintenance of skin flora provided by the investigational product

### Goal

This study aimed to assess the maintenance of skin flora after home use of the product under evaluation.

### Selection of participants

The participants chosen for the study met the following criteria: age group from 18 to 60 years old; gender: female; phototype (fitzpatrick): I to V; intact skin in the tested region; have normal facial skin type; use the same continuous-use drug treatment regimen for at least 4 months; agree not to change the usual of soap (type/brand) used during the study.

### Twenty-nine participants have completed the study.

### Procedure for carrying out evaluations

To assess the skin flora, first, three 2.5 x 4.0 cm sites were marked on the face (forehead, right malar and left malar) of the survey participant. For each site, a dry swab was collected, then the ampoule containing a neutralizing buffer used to facilitate the recovery of bacteria was broken, allowing the collected material to contact the buffer. Each swab was used to assess and count specific microorganisms of the skin flora.

Collection of skin flora was performed at the following times:
*T-14:* 14 days prior to the beginning of use of the assessed product (*wash-out period*): during this period, the survey participants were asked not to apply any cosmetic and/or topical use products on the face.
- *T0*: beginning of use of the assessed product (collection was carried out before the beginning of use).
- *T14:* after 14 days of home use of the assessed product.

For microorganism cultivation, each swab was sown on a specific plate under temperature and growth time conditions established according to the manufacturer's guidelines. Table 3 shows the cultivation information in detail.

**Table 3 - Cultivation Information**

| Microorganism | Culture plate | Cultivation Temperature | Cultivation time |
|---|---|---|---|
| Total Coliforms/ Escherichia coli | 3MTM Petrifilm E. coli / Coliform Count Plate | 35°C±1 | 24 hours |
| *Staphylococcus aureus* | 3MTM Petrifilm Staph Express (STX) System | 35°C±1 | 24 hours |
| Molds and Yeasts | 3MTM Petrifilm Yest and Mold Mount (YM) | 22°C±1 | 3 days (partial count) and 5 days (final count) |

### Results

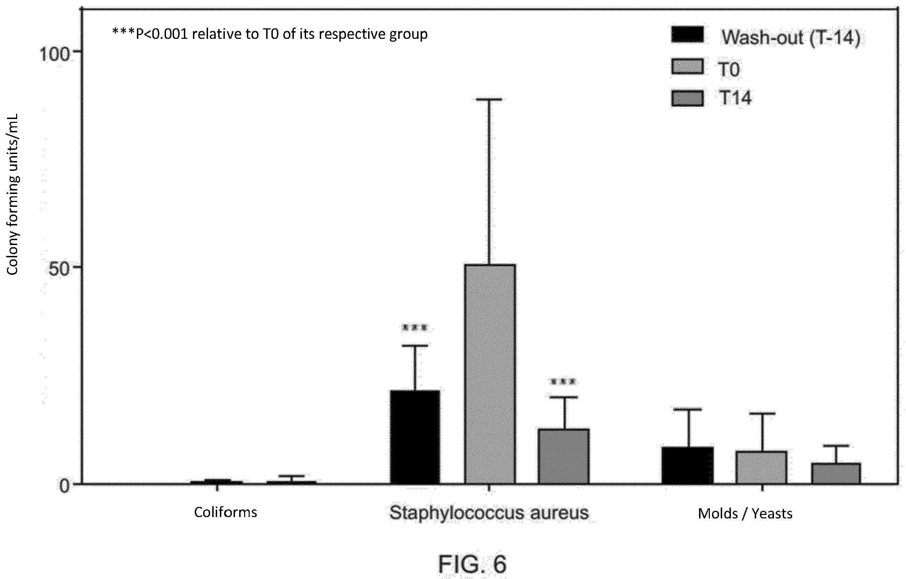

Figure 6 is graph showing the results achieved after counting colony forming units/mL (CFU/mL). As seen, there was no significant change in the number of CFUs/mL of fecal coliforms and Molds/Yeasts.

A significant increase in CFUs/mL of *S. aureus* was observed in the T0 group when compared to the *wash-out* group (P<0.001), which was reversed after 14 days of use of the assessed product (T14).

According to the study and procedures carried out to assess maintenance of the skin flora, so far it has been observed that there were no significant differences in the number of CFUs/mL of fecal coliforms and Molds/Yeasts. On the other hand, a significant increase in the number of CFUs/mL of *S. aureus* was observed in the T0 group, when compared to the *wash-out* group, which was reversed after 14 days of use of the product (T14), reaching values similar to those observed in T-14. Moreover, after 14 days of home use of the assessed product, it was clinically verified that 100% of the research participants evaluated during the study period did not exhibit any characteristic irritating or allergenic skin reactions or any other signs or symptoms of irritating or allergenic skin reactions resulting from of the use of the assessed product.

### Example 4 - Effectiveness analysis of the association

The placebo, as described in table 4, is a traditional moisturizer mainly comprising the following ingredients: water, caprylic, sodium gluconate and sorbitol

**Table 4 - Placebo composition.**

| Compound | Minimum Concentration (%) | Maximum Concentration (%) |
|---|---|---|
| Alpha-isomethyl ionone | 0.002 | 0.005 |
| Ammonium acrylomethyldimethyltaurate copolymer | 0.4 | 2 |
| Water | 43 | 81 |
| Capric/caprylic triglyceride | 1 | 3 |
| Carbomer | 0.3 | 0.6 |
| CI 42090 | 0.00006 | 0.0001 |
| Citronellol | 0.005 | 0.02 |
| *Conobea scoparioides* leaf oil | 0.001 | 0.003 |
| Dimethicone | 7 | 15 |
| Glycerin | 7 | 15 |
| Hydroxyacetophenone | 0.3 | 0.6 |
| Parfum | 0.1 | 0.3 |
| Pentylene glycol | 7 | 15 |
| Phenoxyethanol | 0.5 | 0.8 |
| Polyglyceryl-2 Sesquiisostearate | 0.04 | 0.1 |
| Polymethylsilsesquioxane | 1 | 3 |
| Propanediol | 3 | 7 |
| Sodium carbonate | 0.0003 | 0.0006 |
| Sodium Chloride | 0.00001 | 0.00004 |

| Compound | Minimum Concentration (%) | Maximum Concentration (%) |
|---|---|---|
| Sodium gluconate | 0.05 | 0.15 |
| Sodium hydroxide | 0.07 | 0.15 |
| Sodium Sulfate | 0.000004 | 0.000007 |
| Sorbitol | 0.1 | 0.3 |
| Tocopherol | 0.3 | 0.7 |
| Trilaureth-4 Phosphate | 0.05 | 0.1 |

The results shown below will be compared with "complex I" and "complex II". To illustrate the technical effect of the present invention, complex I comprises the association containing 3% by weight trehalose, 0.5% by weight *Fevillea trilobata* seed oil and 0.25% by weight sodium hyaluronate; complex II comprises a combination containing **% by weight of trehalose,** 0.5% by weight *Fevillea trilobata* seed oil and 0.25% and 2% by weight betaine.

### Results of active hydration assessment

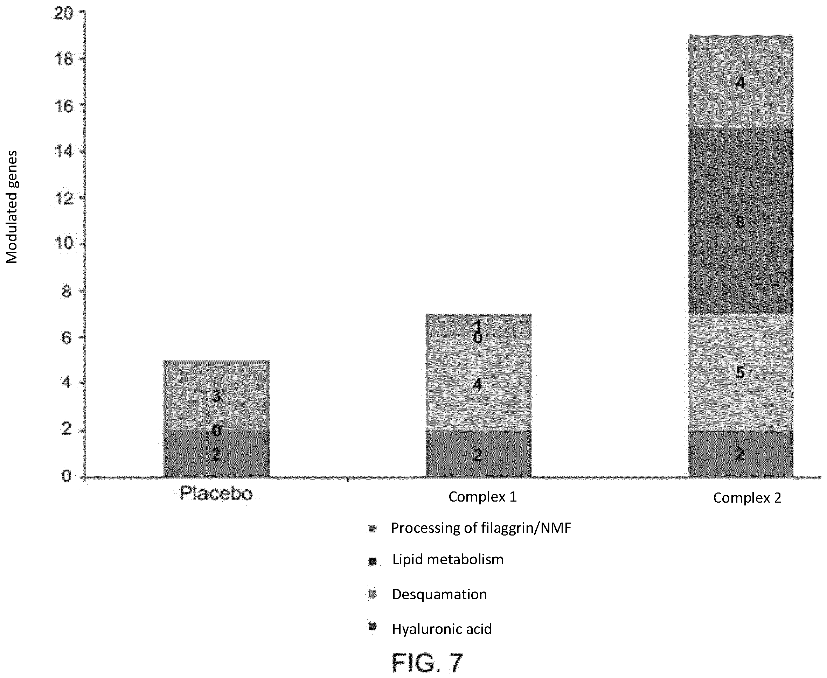

Active hydration for the placebo, complex I and complex II was calculated and demonstrated in Figure 7 by evaluating the expression of genes involved in skin endogenous hydration or self-hydration. The results demonstrate that the placebo composition was not able to provide skin cell renewal; it was not able to stimulate lipids. Complex I had a technical effect, as it promoted a balanced skin desquamation. Complex II had an even superior technical effect, as it stimulated all mechanisms of skin self-hydration, or active hydration, such as balanced desquamation, lipid stimulation and metabolism, NMF stimulation and hyaluronic acid stimulation.

Table 5 below complements the results achieved in the quantification assay of specific markers (or proteins) in view of the skin self-hydration or active hydration mechanisms for the final composition (complex II) and placebo. The results show that the placebo composition was not able to provide skin cell renewal; it was not able to stimulate lipids or NMF and stimulated only one of the essential proteins. However, the cosmetic composition had an even superior technical effect, as it stimulated all mechanisms of skin self-hydration, or active hydration, such as balanced desquamation, lipid stimulation and metabolism, NMF stimulation and stimulation of hyaluronic acid and essential proteins.

**Table 5 - Results of the comparison between placebo and the final composition.**

| Protein confirmation P<0.05 | Placebo | Final composition |
|---|---|---|
| Hyaluronic acid | 19.5% | 28.3% |
| NMF | -17.5% | 22.8% |
| Lipid Synthesis | -29.0%/0.8% | 18.4%/30.1 % |
| Essential proteins | -367.4%/- 17.5%/-29.0%/- 22.3%/0.8% | 435.6%/22.8%/ 18.4%/32.3%/3 0.1%/32.3% |
| Desquamation | -22.3% | 32.3% |

### Results of the screening on the impact on microbiota.

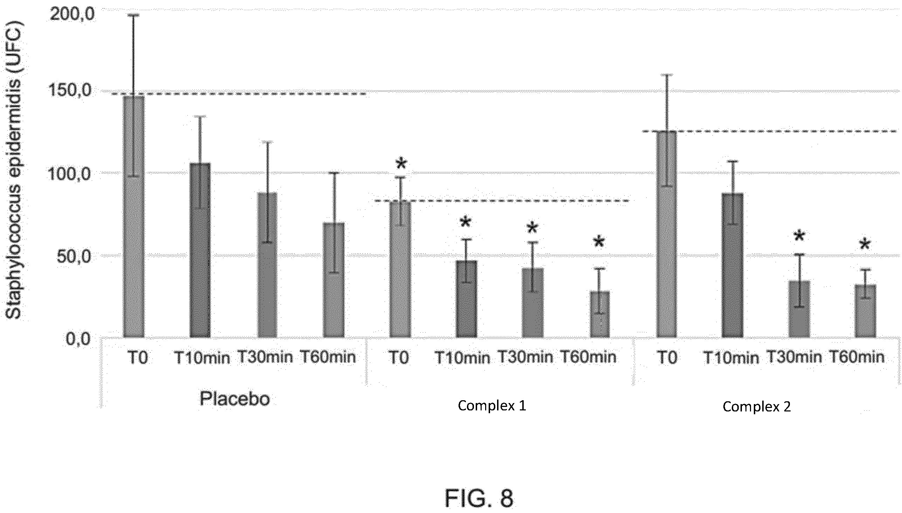

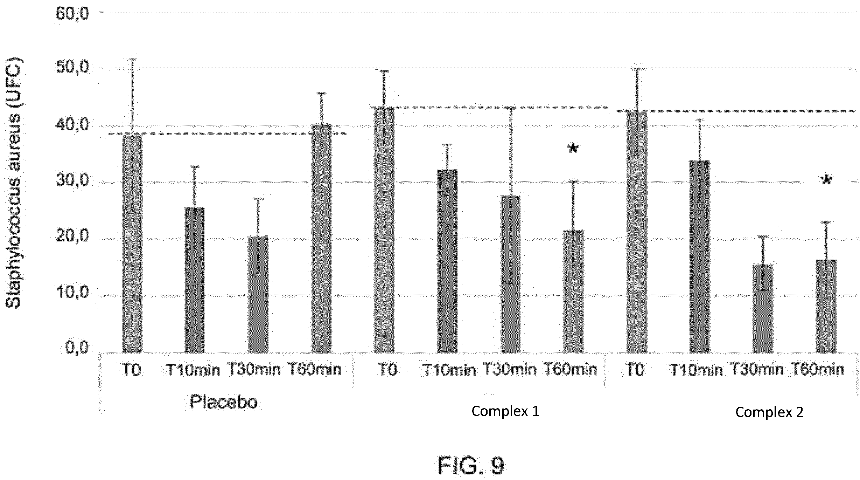

Figures 8 and 9 show the viability kinetics for *S*. *epidermidis and S. aureus.*

In Figure 8, *S*. *epidermidis* viability kinetics is equivalent for the 3 samples. The count has dropped over time, but none of the samples made the presence of the beneficial organism unfeasible.

In figure 9, the placebo favored the recovery of *S. aureus* at T60. This is an unwanted feature, as *S. aureus* is involved in several skin pathologies. This effect did not happen when actives were added to both formulations.

### Sensory consumer research

A sensory consumer research was carried out. This research was carried out quantitatively based on the home use of the product by 120 women aged 18 to 45 years, class AB. These women had all skin types and were users of facial moisturizers at least five times a week.

The study lasted for 07 days and was carried out in two moments:
1^{st} day: first contact of the participants with the product. They received instructions for use and applied the facial moisturizer. After using the product, they answered an immediate evaluation questionnaire and received the product for use for 07 days.
7^{th} day: after 7 days of use, all volunteers returned to answer the final questionnaire on the facial moisturizer.

The research results are shown in table 6.

**Table 6 - Agreement indices (Top 2 Box), immediate assessment - 1^{st} day.**

| Agreement analysis (Immediate assessment - 1^{st} day) | TOP 2 BOX (%) |
|---|---|
| It makes my skin smooth | 96 |
| It makes my skin velvety | 85 |
| It moisturizes my skin in a single application | 82 |
| It replenishes skin moisture instantly | 83 |
| It makes my skin hydrated | 94 |
| It makes my skin rebalanced | 78 |
| It revitalizes my skin | 84 |
| It renews my skin | 86 |
| It gives my skin a renewal feel | 90 |
| It renews my skin, making it more beautiful and healthier | 82 |
| It rests my skin | 87 |
| It makes my skin shiny | 68 |
| It makes my skin radiant | 73 |
| It refreshes my skin | 88 |
| It strengthens my skin | 84 |
| It makes my skin free from signs of dryness | 90 |
| It quickly penetrates the skin | 85 |
| It suits my skin type | 88 |
| It has a light formula | 93 |
| It has refreshing formula | 90 |
| It has an ultra-hydrating formula | 83 |
| It makes my skin oily | 15 |
| It makes my skin sticky | 16 |

**Table 7 - Agreement indices (Top 2 Box), Assessment on the 7^{th} day.**

| Agreement analysis (Assessment - 7^{th} day) | TOP 2 BOX (%) |
|---|---|
| It makes my skin smooth | 96 |
| It makes my skin velvety | 94 |
| It moisturizes my skin in a single application | 88 |
| It replenishes skin moisture instantly | 94 |
| It makes my skin hydrated | 97 |
| It makes my skin rebalanced | 92 |
| It revitalizes my skin | 94 |
| It renews my skin | 94 |
| It gives my skin a renewal feel | 93 |
| It renews my skin, making it more beautiful and healthier | 94 |
| It rests my skin | 93 |
| It makes my skin shiny | 83 |
| It makes my skin radiant | 88 |
| It refreshes my skin | 92 |
| It strengthens my skin | 91 |
| It makes my skin free from signs of dryness | 92 |
| It quickly penetrates the skin | 86 |
| It suits my skin type | 92 |
| It has a light formula | 93 |
| It has refreshing formula | 92 |
| It has an ultra-hydrating formula | 93 |
| It makes my skin oily | 19 |
| It makes my skin sticky | 23 |

Those skilled in the art will value the knowledge presented herein and may reproduce the invention in the disclosed embodiments and other variants, as covered by the scope of the appended claims.

## Claims

1. Topical cosmetic composition, **characterized in that** it comprises the association of *Fevillea trilobata* seed crude oil, trehalose, sodium hyaluronate, betaine and cosmetically acceptable excipients.

2. The composition according to claim 1, **characterized in that** it comprises:
from 0.1 to 3% by weight *Fevillea trilobata* crude seed oil;
from 1 to 4% by weight trehalose;
from 0.1 to 2% by weight sodium hyaluronate;
from 1 to 5% by weight betaine;
and from 86% to 97.8% by weight of cosmetically acceptable excipients.

3. The cosmetic composition, according to any one of claims 1 to 2, **characterized in that** the cosmetically acceptable excipients are selected from at least one of the classes: viscosity controllers, anti-foaming agents, emollients, skin conditioners, skin protectors, denaturants, perfumes, moisturizers, opacifiers, emulsifiers, preservatives, antioxidants, vegetable oils, emulsion stabilizers, plasticizers, chelators, buffers and colorants.

4. The cosmetic composition, according to claim 3, **characterized in that** the cosmetically acceptable excipients are selected from: the solvent can be water, propanediol and pentylene glycol, or a combination thereof; the anti-foaming agent can be dimethicone; the emollient can be dimethicone, vegetable oils and tocopherol, or a combination thereof; the skin conditioner can be dimethicone, pentylene glycol, glycerin, betaine, capric/caprylic triglyceride, tocopherol, sodium hyaluronate, sorbitol, sodium gluconate, or a combination thereof; the skin protector can be dimethicone, glycerin or a combination thereof; the viscosity controller can be propanediol, glycerin, betaine, carbomer, ammonium acryloyldimethyltaurate copolymer, sodium chloride, sodium sulfate or combination thereof; the denaturant can be glycerin, sodium hydroxide, eugenol or a combination thereof; the humectant can be glycerin, trehalose, betaine, sodium hyaluronate, sorbitol or a combination thereof; fragrances and perfumes can be used; opacifier can be polymethylsilsesquioxane; the emulsifier can be trilaureth-4 phosphate, polyglyceryl-2 sesqui-isostearate or combination thereof; the preservative can be phenoxyethanol or hydroxyacetophenone or a combination thereof; the antioxidant can be tocopherol, hydroxyacetophenone or combination thereof; the emulsion stabilizer can be carbomer; the plasticizer can be sorbitol; the chelator can be sodium gluconate; and the buffering agent can be sodium hydroxide; sodium carbonate or a combination thereof.

5. The cosmetic composition, according to any one of claims 1 to 4, **characterized in that** it is an emulsion, cream, creamy emulsion, gels, cream gels, aqua gels, acqua gels and serums.

6. The cosmetic composition according to any one of claims 1 to 5, **characterized in that** it is an acqua gel.

7. Use of the topical cosmetic composition, as defined in any one of claims 1 to 6, **characterized in that** it is to hydrate the body or face skin.

8. Use according to claim 7, **characterized in that** it is to hydrate the face skin.

9. Use of the topical cosmetic composition as defined in any one of claims 1 to 6, **characterized in that** it is to preserve the skin natural microbiota.

10. A non-therapeutic method to hydrate and/or preserve the skin natural microbiota **characterized in that** it comprises the topical application of the cosmetic composition, as defined in any one of claims 1 to 6.

## Patentansprüche

1. Topische kosmetische Zusammensetzung, **dadurch gekennzeichnet, dass** sie die Verbindung aus Rohöl aus Samen von *Fevillea trilobata*, Trehalose, Natriumhyaluronat, Betain und kosmetisch akzeptablen Hilfsstoffen umfasst.

2. Zusammensetzung nach Anspruch 1, **dadurch gekennzeichnet, dass** sie umfasst:
von 0,1 bis 3 Gew.-% an Rohöl aus Samen von Fevillea trilobata;
von 1 bis 4 Gew.-% an Trehalose;
von 0,1 bis 2 Gew.-% an Natriumhyaluronat;
von 1 bis 5 Gew.-% an Betain;
und von 86 Gew.-% bis 97,8 Gew.-% an kosmetisch akzeptablen Hilfsstoffen.

3. Kosmetische Zusammensetzung, nach einem der Ansprüche 1 bis 2, **dadurch gekennzeichnet, dass** die kosmetisch akzeptablen Hilfsstoffe aus mindestens einer der Klassen ausgewählt sind: Viskositätsregler, Antischaummittel, Emollienzien, Hautkonditioniermittel, Hautschutzmittel, Vergällungsmittel, Parfümstoffe, Feuchtigkeitsspender, Opazitätsmittel, Emulgatoren, Konservierungsmittel, Antioxidantien, Pflanzenöle, Emulsionsstabilisatoren, Weichmacher, Chelatbildner, Puffer und Farbstoffe.

4. Kosmetische Zusammensetzung, nach Anspruch 3, **dadurch gekennzeichnet, dass** die kosmetisch akzeptablen Hilfsstoffe ausgewählt sind aus: dem Lösungsmittel, das Wasser, Propandiol und Pentylenglykol oder eine Kombination davon sein kann; dem Entschäumungsmittel, das Dimethicon sein kann; dem Emolliens, das Dimethicon, Pflanzenöle und Tocopherol oder eine Kombination davon sein kann; dem Hautkonditioniermittel, das Dimethicon, Pentylenglykol, Glycerin, Betain, Caprin-/Caprylsäuretriglycerid, Tocopherol, Natriumhyaluronat, Sorbitol, Natriumgluconat oder eine Kombination davon sein kann; dem Hautschutzmittel, das Dimethicon, Glycerin oder eine Kombination davon sein kann; dem Viskositätsregler, der Propandiol, Glycerin, Betain, Carbomer, Ammoniumacryloyldimethyltaurat-Copolymer, Natriumchlorid, Natriumsulfat oder eine Kombination davon sein kann; dem Vergällungsmittel, das Glycerin, Natriumhydroxid, Eugenol oder eine Kombination davon sein kann; dem Feuchthaltemittel, das Glycerin, Trehalose, Betain, Natriumhyaluronat, Sorbitol oder eine Kombination davon sein kann; Duftstoffen und Parfümstoffen, die verwendet werden können; dem Opazitätsmittel, das Polymethylsilsesquioxan sein kann; dem Emulgator, der Trilaureth-4-Phosphat, Polyglyceryl-2-Sesqui-Isostearat oder eine Kombination davon sein kann; dem Konservierungsmittel, das Phenoxyethanol oder Hydroxyacetophenon oder eine Kombination davon sein kann; dem Antioxidans, das Tocopherol, Hydroxyacetophenon oder eine Kombination davon sein kann; dem Emulsionsstabilisator, der Carbomer sein kann; dem Weichmacher, der Sorbitol sein kann; dem Chelatbildner, der Natriumgluconat sein kann; und dem Puffer, der Natriumhydroxid; Natriumcarbonat oder eine Kombination davon sein kann.

5. Kosmetische Zusammensetzung, nach einem der Ansprüche 1 bis 4, **dadurch gekennzeichnet, dass** sie eine Emulsion, Creme, cremige Emulsion, Gels, Cremegels, Aqua-Gels, Acqua-Gels und Seren ist.

6. Kosmetische Zusammensetzung nach einem der Ansprüche 1 bis 5, **dadurch gekennzeichnet, dass** sie ein Acqua-Gel ist.

7. Verwendung der topischen kosmetischen Zusammensetzung nach einem der Ansprüche 1 bis 6, **dadurch gekennzeichnet, dass** sie dazu dient, die Haut des Körpers oder des Gesichts zu hydratisieren.

8. Verwendung nach Anspruch 7, **dadurch gekennzeichnet, dass** sie dazu dient, die Haut des Gesichts zu hydratisieren.

9. Verwendung der topischen kosmetischen Zusammensetzung nach einem der Ansprüche 1 bis 6, **dadurch gekennzeichnet, dass** sie dazu dient, die natürliche Mikrobiota der Haut aufrechtzuerhalten.

10. Nichttherapeutisches Verfahren zum Hydratisieren und/oder Aufrechterhalten der natürlichen Mikrobiota der Haut
**dadurch gekennzeichnet, dass** es die topische Anwendung der kosmetischen Zusammensetzung nach einem der Ansprüche 1 bis 6 umfasst.

## Revendications

1. Composition cosmétique topique, **caractérisée en ce qu'**elle comprend l'association d'huile brute de graine de *Fevillea trilobata*, de tréhalose, d'hyaluronate de sodium, de bétaïne et d'excipients cosmétiquement acceptables.

2. Composition selon la revendication 1, **caractérisée en ce qu'**elle comprend :
de 0,1 % en poids à 3 % en poids d'huile brute de graine de Fevillea trilobata ;
de 1 % en poids à 4 % en poids de tréhalose ;
de 0,1 % en poids à 2 % en poids d'hyaluronate de sodium ;
de 1 % en poids à 5 % en poids de bétaïne ;
et de 86 % en poids à 97,8 % en poids d'excipients cosmétiquement acceptables.

3. Composition cosmétique selon l'une quelconque des revendications 1 à 2, **caractérisée en ce que** les excipients cosmétiquement acceptables sont choisis parmi au moins l'une des classes suivantes : régulateurs de viscosité, agents antimousse, émollients, conditionneurs cutanés, protecteurs cutanés, dénaturants, parfums, agents hydratants, opacifiants, émulsifiants, conservateurs, antioxydants, huiles végétales, stabilisants d'émulsion, plastifiants, agents chélatants, agents tampons et colorants.

4. Composition cosmétique selon la revendication 3, **caractérisée en ce que** les excipients cosmétiquement acceptables sont choisis parmi : le solvant peut être l'eau, le propanediol et le pentylène glycol, ou une combinaison de ceux-ci ; l'agent antimousse peut être la diméthicone ; l'émollient peut être la diméthicone, les huiles végétales et le tocophérol, ou une combinaison de ceux-ci ; le conditionneur cutané peut être la diméthicone, le pentylène glycol, la glycérine, la bétaïne, le triglycéride caprique/caprylique, le tocophérol, l'hyaluronate de sodium, le sorbitol, le gluconate de sodium, ou une combinaison de ceux-ci ; le protecteur cutané peut être la diméthicone, la glycérine, ou une combinaison de celles-ci ; le régulateur de viscosité peut être le propanediol, la glycérine, la bétaïne, le carbomère, le copolymère d'acryloyldiméthyltaurate d'ammonium, le chlorure de sodium, le sulfate de sodium, ou une combinaison de ceux-ci ; le dénaturant peut être la glycérine, l'hydroxyde de sodium, l'eugénol, ou une combinaison de ceux-ci ; l'humectant peut être la glycérine, le tréhalose, la bétaïne, l'hyaluronate de sodium, le sorbitol, ou une combinaison de ceux-ci ; des fragrances et parfums peuvent être utilisés ; l'opacifiant peut être le polyméthylsilsesquioxane ; l'émulsifiant peut être le phosphate de trilaureth-4, le polyglycéryl-2 sesquiisostéarate, ou une combinaison de ceux-ci ; le conservateur peut être le phénoxyéthanol ou l'hydroxyacétophénone, ou une combinaison de ceux-ci ; l'antioxydant peut être le tocophérol, l'hydroxyacétophénone, ou une combinaison de ceux-ci ; le stabilisant d'émulsion peut être le carbomère ; le plastifiant peut être le sorbitol ; l'agent chélatant peut être le gluconate de sodium ; et l'agent tampon peut être l'hydroxyde de sodium, le carbonate de sodium, ou une combinaison de ceux-ci.

5. Composition cosmétique selon l'une quelconque des revendications 1 à 4, **caractérisée en ce qu'**elle est une émulsion, une crème, une émulsion crémeuse, des gels, des gels-crèmes, des gels aqua, des gels acqua et des sérums.

6. Composition cosmétique selon l'une quelconque des revendications 1 à 5, **caractérisée en ce qu'**elle est un gel acqua.

7. Utilisation de la composition cosmétique topique, telle que définie dans l'une quelconque des revendications 1 à 6, **caractérisée en ce qu'**elle est destinée à hydrater la peau du corps ou du visage.

8. Utilisation selon la revendication 7, **caractérisée en ce qu'**elle est destinée à hydrater la peau du visage.

9. Utilisation de la composition cosmétique topique telle que définie dans l'une quelconque des revendications 1 à 6, **caractérisée en ce qu'**elle est destinée à préserver le microbiote naturel de la peau.

10. Procédé non thérapeutique pour hydrater et/ou préserver le microbiote naturel de la peau
**caractérisé en ce qu'**il comprend l'application topique de la composition cosmétique, telle que définie dans l'une quelconque des revendications 1 à 6.
